# FASCICULE DE BREVET EUROPEEN

(11) **EP 1 778 097 B1**
(45) Date de publication et mention de la délivrance du brevet: **08.09.2010**
(21) Numéro de dépôt: 05793255.0
(22) Date de dépôt: 27.07.2005
(51) Int. Cl.: A61B 17/04, A61B 17/08

(54) **INSTRUMENT ET DISPOSITIF D'ANCRAGE PARIETAL NOTAMMENT POUR CHIRURGIE LAPAROSCOPIQUE OU COELIOSCOPIQUE**
PARIETALES VERANKERUNGSWERKZEUG UND VORRICHTUNG, INSBESONDERE FÜR LAPAROSKOPISCHE UND COLOSKOPISCHE CHIRURGIE
PARIETAL ANCHORING TOOL AND DEVICE, IN PARTICULAR FOR LAPAROSCOPIC OR COELIOSCOPIC SURGERY

(30) Priorité: 27.07.2004 FR 0408299
(43) Date de publication de la demande: 02.05.2007
(73) Titulaire: Messas, Aurel, 75004 Paris (FR)
(72) Inventeur: Messas, Aurel, 75004 Paris (FR)
(74) Mandataire: Jacobson, Claude
(86) Numéro de dépôt international: PCT/FR2005/001967
(87) Numéro de publication internationale: WO 2006/018546

(56) Documents cités:
- WO-A-95/26170
- US-A- 3 274 658
- US-A- 5 954 057
- US-A1- 2003 167 055
- US-A1- 2004 116 949

## Description

L'invention concerne un instrument d'ancrage pariétal d'un organe d'un être vivant, destiné à être utilisé pour exposer une structure anatomique au cours d'une opération chirurgicale, notamment en laparoscopie ou en coelioscopie.

Pour réaliser une intervention chirurgicale en laparoscopie, ou en coelioscopie, il est connu de gonfler artificiellement l'abdomen de l'être humain ou animal à opérer, puis d'introduire des trocarts dans la paroi abdominale pour créer des ports d'entrées étanches vers l'espace opératoire.

La région anatomique à opérer est visionnée par le chirurgien à l'aide d'une caméra spécifique introduite dans l'espace opératoire au travers d'un trocart. L'exposition de la région anatomique à opérer est réalisée par un ou deux aides opératoires qui écartent les organes localisés devant la région à opérer, à l'aide de pinces, spatules, ou crochets. Ainsi, il est nécessaire d'avoir un chirurgien et un ou deux aides opératoires pour opérer un patient en laparoscopie.

Un aide opératoire peut, pour certaines opérations, être remplacé par un bras robotisé. Cependant, ces bras robotisés ont un coût d'achat élevé, nécessitent une maintenance et sont susceptibles de tomber en panne.

Un but de l'invention est de proposer un instrument à faible coût à même de remplacer un aide opératoire ou un bras robotisé pour réaliser une intervention chirurgicale en laparoscopie et en coelioscopie.

Par ailleurs, l'exposition d'une structure anatomique est obtenue à l'aide de pinces chirurgicales traversant la paroi abdominale au travers de trocarts implantés dans celle-ci. Ainsi, cinq à sept trocarts peuvent être implantés dans la paroi abdominale. Or, l'implantation de chaque trocart nécessite une incision dans la paroi abdominale,

Un autre but de l'invention est de limiter le nombre de trocarts implantés de manière à réduire le nombre de cicatrices résultant d'une opération.

A cet effet, l'invention a pour objet un instrument d'ancrage pariétal d'un organe humain ou animal, tel que défini dans la revendication 1.

Des modes particuliers de réalisation de l'invention sont décrits dans les revendications 2 à 6.

L'invention a également pour objet un dispositif d'ancrage pariétal d'un organe d'un être vivant au cours d'une opération chirurgicale en laparoscopie ou en coelioscopie, **caractérisé en ce qu**'il comprend :
- au moins un élément de traction propre à traverser la paroi abdominale dudit être vivant ;
- des moyens pour fixer extérieurement ledit élément de traction à la paroi abdominale dudit être vivant ; et
- un instrument d'ancrage pariétal tel que défini ci-dessus, ledit élément de traction étant propre à être fixé d'une part aux moyens d'attache dudit instrument localisés à l'intérieur de la paroi abdominale, et d'autre part audit moyen de fixation à l'extérieur de la paroi abdominale pour former un point d'ancrage fixe de l'organe maintenu suspendu par l'instrument d'ancrage et l'élément de traction.

On connaît l'état de la technique suivant :
- US 2003/0167055 A1, qui décrit une pince chirurgicale qui n'est pas un instrument de laparoscopie ou de coelioscopie, ni un instrument d'ancrage pariétal. Cet instrument vise à suspendre le coeur d'un patient en fixant l'extrémité libre de l'élément de traction à travers une incision du thorax, en chirurgie conventionnelle.
- US-A-3 274 658 décrit une pince chirurgicale classique.
- WO 95/26 170 A décrit un crochet de microchirurgie qui n'est pas adapté pour être manipulé par une pince (et donc à une seule main) mais qui nécessite le coulissement d'un manchon 5 le long du crochet, et donc l'intervention de deux mains.
- US-A-5 954 057 décrit un instrument qui n'est nullement un instrument d'ancrage pariétal d'un organe, mais un instrument d'ancrage d'un organe à un os. Plus particulièrement, dans ce document, il ne s'agit pas d'exposer temporairement un organe au cours d'une intervention chirurgicale, mais de fixer un organe de façon définitive dans une nouvelle position. En d'autres termes, l'instrument reste en permanence dans le corps comme un implant.

L'invention sera mieux comprise à la lecture de la description qui va suivre, donnée uniquement à titre d'exemple et faite en se référant aux dessins annexés, sur lesquels :
- la figure 1 est une vue schématique de côté d'un instrument d'ancrage pariétal selon un mode de réalisation de l'invention ;
- la figure 2 est une vue de dessus de l'instrument représenté sur la figure 1 ; et
- les figures 3 à 6 sont des vues schématiques en coupé d'un bassin abdominal au cours de différentes étapes d'un procédé d'ancrage pariétal par un instrument d'ancrage selon l'invention.

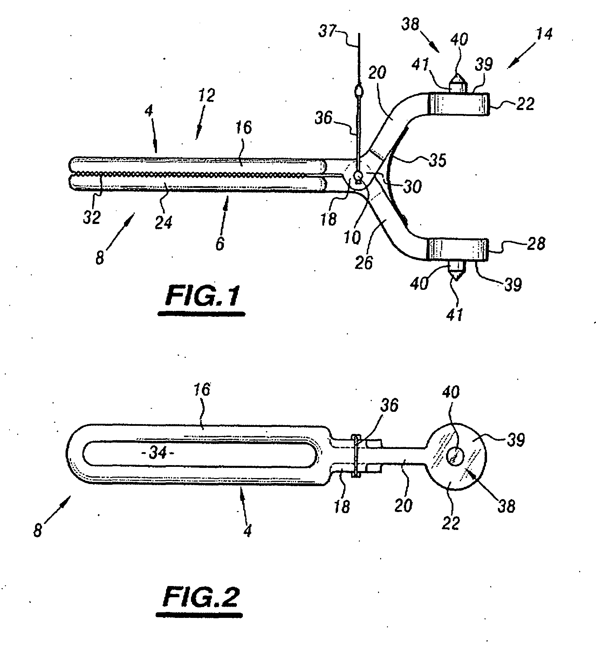

L'instrument d'ancrage pariétal illustré schématiquement sur les figures 1 et 2 est une pince. Celle-ci comprend un premier 4 et un second 6 éléments longitudinaux formés chacun d'un seul tenant dans une matière métallique ou plastique. Ils présentent une longueur d'environ deux centimètres.

Comme visible sur la figure 1, le premier 4 et le second 6 éléments longitudinaux sont articulés l'un à l'autre, au niveau de leur partie médiane, pour constituer une pince 8. Cette pince 8 comprend, de part et d'autre, de l'axe d'articulation 10, une mâchoire 12 d'accrochage d'un organe et des moyens 14 de préhension de l'instrument et de commande de l'ouverture de la mâchoire 12. Les éléments longitudinaux 4, 6 sont aptes à pivoter autour de l'axe 10, entre une position de fermeture et une position d'ouverture de la pince 8.

Plus précisément, le premier élément longitudinal 4 comporte successivement un méplat ajouré 16, une chape 18, une barrette de liaison 20 et un disque d'appui 22. Le méplat 16 et le disque 22 s'étendent dans des plans parallèles, non coplanaires. La chape 18 est formée dans le prolongement du méplat 16, de manière adjacente à la barrette 20,

Le second élément longitudinal 6 est de forme similaire au premier élément 4. Il comprend ainsi également un méplat 24, une barrette de liaison 26 et un disque d'appui 28. Cependant, une saillie convexe 30 remplace la chape 18. Cette saillie convexe 30 est engagée dans la chape 18. L'axe 10 traverse la saillie 30 et la chape 18 pour articuler le premier élément 4 au second élément 6.

Les deux méplats 16 et 24 constituent la mâchoire 12 d'accrochage d'un organe de la pince. Ils présentent sur leurs surfaces en regard l'une de l'autre des stries 32 anti-dérapantes pour faciliter l'accrochage des organes humains ou animaux. Les méplats 16 et 24 comprennent de plus, sur toute leur longueur, une ouverture 34 oblongue et traversante.

Un ressort 35 de maintien dé la mâchoire 12 dans une position de fermeture est monté entre les barrettes 20 et 26. Ce ressort 35 est apte à exercer une force de pression propre à rassembler les méplats 16 et 24. Ainsi, au repos, lorsque aucune pression n'est exercée sur la surface externe des disques d'appui 22 et 28, la pince 8 est maintenue dans une position de fermeture ou de pincement d'un organe. Le ressort 35 est formé par une lame métallique fixée à la barrette 26 et en appui contre la barrette 20. Cette lame est apte à exercer une force de résistance à l'ouverture de la pince.

Les disques d'appui 22, 28 et les barrettes de liaison 20, 26 forment les moyens 14 de préhension et de commande de l'ouverture de la mâchoire 12. Ils permettent de manipuler et de déplacer l'instrument d'ancrage pendant l'opération chirurgicale.

L'instrument d'ancrage comprend également un arceau 36 d'attache d'au moins un élément de traction 37 à la pince 8. Cet élément de traction est de préférence souple. Il est par exemple constitué par un fil, une bande, une lanière etc...Le fil de traction 37 est propre à être fixé à la paroi abdominale de l'être vivant à opérer pour soulever et maintenir en suspension un organe accroché par la mâchoire 12. Le fil peut également porter un organe en un point situé entre la pince et le point de sortie pariétal du fil, comme cela sera décrit ci-après dans le procédé d'utilisation de l'instrument d'ancrage selon l'invention.

Le fil de traction 37 est solidarisé à l'arceau 36 par exemple par soudure souple. Cet arceau est solidaire à chaque extrémité de l'axe 10. L'arceau 36 est composé d'un matériau semi-élastique de type matière plastique ou caoutchouc, de sorte qu'il se déforme en fonction de la direction de traction du fil.

L'arceau 36 est positionné à une certaine distance de la mâchoire 12 et des moyens 14 de préhension et de commande de cette mâchoire, pour que le fil 37 ne s'enroule pas autour de ceux-ci. Par ailleurs, l'arceau 36 fait saillie par rapport à la mâchoire 12 et aux moyens 14 pour faciliter l'accrochage d'un fil de traction supplémentaire au cours de l'opération chirurgicale.

Les moyens 14 comprennent également des protubérances 38 en saillie sur la surface 39 externe de chaque disque d'appui 22, 28 pour faciliter la préhension, la manipulation et la commande d'ouverture de la pince 8 à partir d'une pince chirurgicale classique. Chaque protubérance 38 comprend une partie d'extrémité 40 tronconique et une base 41 cylindrique solidaire du disque d'appui 22, 28. La base 41 a un diamètre légèrement inférieur au diamètre des fenêtres d'extrémité des pinces chirurgicales classiques pour permettre leur insertion dans celles-ci.

L'instrument d'ancrage pariétal représenté sur les figures 1 et 2 est un exemple de réalisation de l'invention nullement limitatif. La forme de cet instrument varie en fonction du type d'organe à saisir et de son caractère atromatique.

Selon une autre variante non représentée du mode de réalisation de l'invention, les moyens d'attache 36 du fil de traction sont formés par un orifice traversant l'instrument d'ancrage. Le fil est propre à être introduit dans cet orifice puis à être attaché autour d'une paroi de l'instrument. Cet orifice peut par exemple, être pratiqué au travers de la saillie convexe 30 et de la chape 18, ou au travers d'une barrette de liaison 20, 26 de la pince 8.

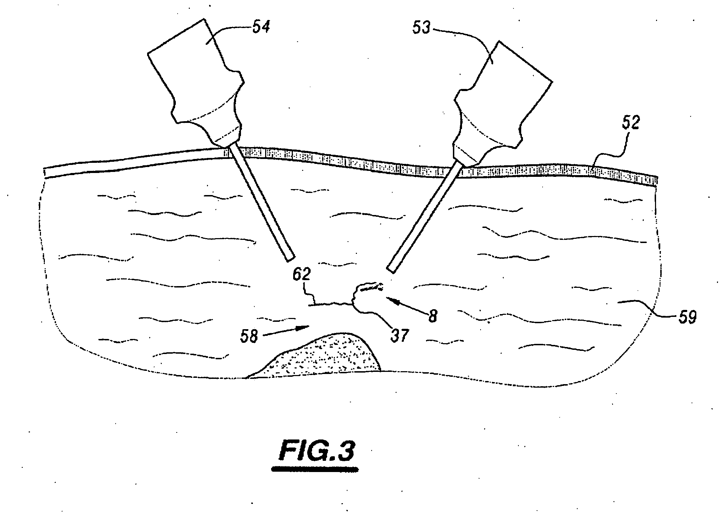

Les figures 3 à 6 illustrent un procédé d'ancrage pariétal réalisé à l'aide d'un instrument d'ancrage selon le premier mode de réalisation de l'invention.

La figure 3 représente une vue schématique en coupe d'un espace opératoire délimité par une paroi abdominale 52.

Pendant une première étape de l'opération, deux trocarts 53 et 54 sont introduits dans la paroi 52.

Puis, au cours d'une deuxième étape de l'opération, un dispositif d'ancrage 58 est amené, via le trocart 53, dans l'espace opératoire 59 de l'abdomen. Ce dispositif 58 comprend une pince d'ancrage 8, un fil de traction 37 et une aiguille 62. Une extrémité du fil 37 est fixée à l'arceau 36 de la pince 8. L'autre extrémité du fil 37 est solidaire de l'aiguille 62.

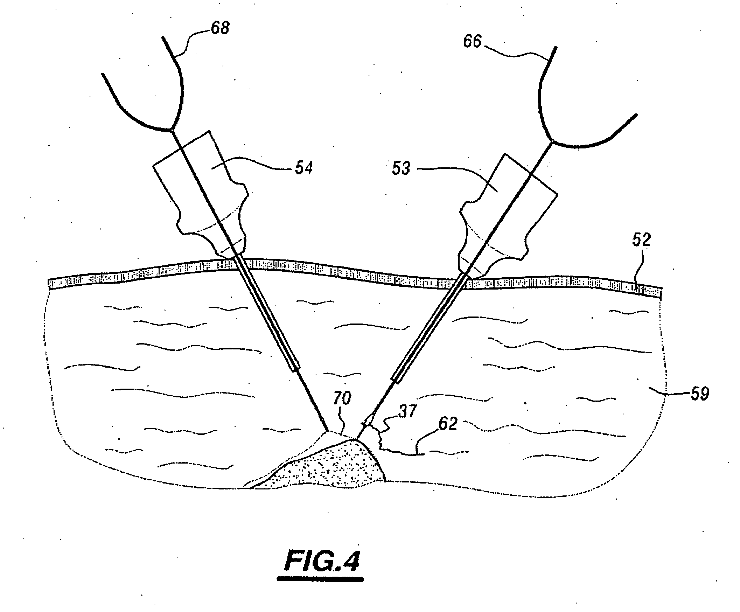

Selon une troisième étape de l'opération, illustrée sur la figure 4, le chirurgien introduit dans chaque trocart 53 et 54 une pince chirurgicale classique 66, 68. Puis il écarte un organe disposé devant la région anatomique à opérer, à l'aide d'une pince 68. Cette pince saisit un organe 70 pour exposer la zone à opérer.

Pour maintenir cet organe 70 à l'écart de la zone à opérer dans une position statique, le chirurgien ou l'aide opératoire insère les protubérances 38 des moyens 14 de préhension et de commande de la pince 8 dans les fenêtres des extrémités de la pince 66 ou 68 libre, commande l'ouverture de la mâchoire 12 par les moyens 14 de préhension et de commande, et accroche dans les mâchoires 12 l'organe 70.

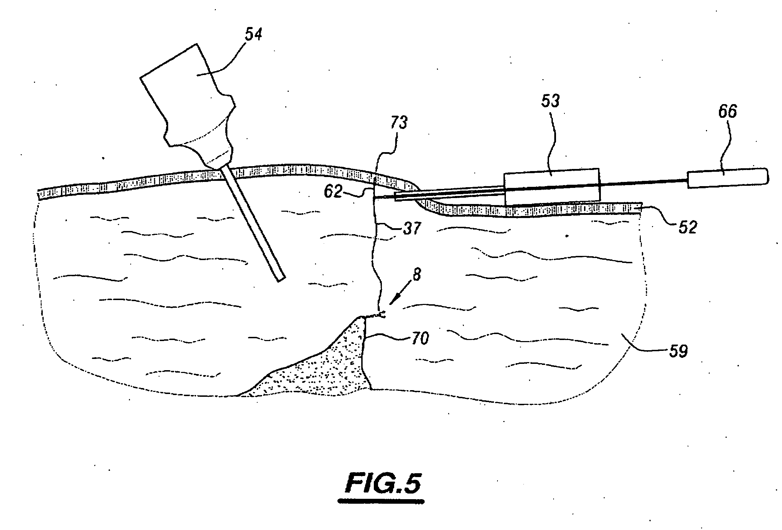

Selon une quatrième étape, illustrée sur la figure 5, le chirurgien transperce la paroi abdominale 52 à l'aide de l'aiguille 62, manipulée par la pince 66, en un point d'ancrage choisi 73. L'aiguille 62 et une partie du fil 37 sont ainsi amenées à l'extérieur de l'espace opératoire 59. Lors de cette opération, le fil 37 tracte l'organe 70 et la pince 8 en suspension de manière à ce que l'organe 70 s'éloigne de la zone à opérer.

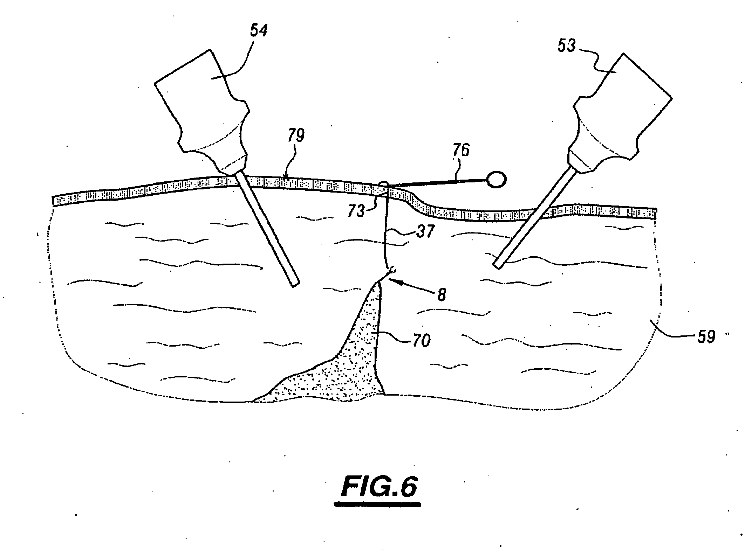

Selon.une cinquième étape, illustrée sur la figure 6, le fil 37 est fixé à l'extérieur de la cavité abdominale 59 à l'aide d'un moyen de fixation 76 comprenant par exemple une pince. Le fil 37, fixé à la paroi abdominale 52, constitue ainsi un point 73 d'ancrage fixe de l'organe 70. L'organe 70 est maintenu écarté de la zone à opérer, sans être tenu par un aide opératoire ou un bras robotisé. Le fil 37 maintient une traction constante sur l'organe 70 sans le transpercer.

En variante, le fil est attaché à l'intérieur de la cavité abdominale à un système d'ancrage intraabdominal transpariétal c'est-à-dire à un système de fixation intrapariétal relié à un système extrapariétal qui ne passe pas au travers d'un trocart. Dans ce cas, plusieurs fils de traction peuvent avantageusement être attachés au système de fixation intrapariétal.

Avantageusement, la position de l'organe 70 peut être réajustée durant l'intervention par simple traction du fil 37 en dehors de la paroi (ou inversement) et repositionnement du moyen de fixation 76.

Plusieurs instruments d'ancrage 8 de ce type peuvent être utilisés simultanément durant une intervention pour optimiser l'exposition et libérer les pinces chirurgicales 66 et 68 passant par les trocarts 53, 54, pour leur permettre de jouer un rôle dynamique durant l'opération.

Avantageusement, l'instrument d'ancrage 8 peut être manipulé et déplacé durant l'intervention, afin de l'accrocher à un autre organe dans la cavité abdominale tout en maintenant le premier point 73 d'ancrage fixe sur la paroi abdominale 52.

Parallèlement, le point 73 d'ancrage de l'organe peut également être déplacé pour obtenir un autre angle de vue sur la zone à opérer, tout en maintenant l'organe 70 dans la mâchoire 12. Par exemple, lorsque le chirurgien veut orienter l'organe 70 selon une autre direction, il introduit dans l'espace opératoire 59 un second fil, non représenté, solidaire de l'aiguille 62. Il fixe l'extrémité de ce second fil à l'arceau 36 de la pince 8. Puis, il coupe le premier fil de traction 37 préalablement attaché. Le second fil est propre à traverser la paroi 50 à l'aide de l'aiguille 62, en un second point d'ancrage 79 différent du premier point d'ancrage 73.

Ainsi, l'instrument d'ancrage pariétal permet de maintenir un organe 70 dans une certaine position pendant une durée choisie à un premier point d'ancrage 73 puis de déplacer cet organe 70 et de le maintenir suspendu selon une orientation différente à un second point d'ancrage 79.

L'instrument d'ancrage pariétal permet donc de faciliter l'exposition d'une zone à opérer sans imposer de trocart supplémentaire et sans nécessiter une main supplémentaire pour exercer une action.

Avantageusement, en diminuant le nombre de trocarts implantés, on diminue le nombre d'incisions dans la paroi abdominale et on accélère le rétablissement du patient, en diminuant la durée de convalescence post opératoire.

Cet instrument d'ancrage permet de diminuer le nombre d'aides opératoires assistant le chirurgien au cours de l'opération, de sorte que, en cas d'hémorragie ou d'autre incident, un aide opératoire dispose d'une main supplémentaire pour agir et traiter l'incident.

Avantageusement, l'extrémité préhensive de la pince 8 peut être manipulée par des instruments coelioscopiques classiques.

Pour récapituler, le procédé d'ancrage pariétal d'un organe d'un être vivant pour exposer une structure anatomique à traiter au cours d'une opération chirurgicale, comporte :
- une première étape d'insertion d'un trocart 53, 54 dans la paroi abdominale 52 d'un être humain ou animal ;
- une deuxième étape d'introduction dans la cavité abdominale 59 d'un instrument 8 d'ancrage pariétal, d'une aiguille 62 et d'au moins un fil 37 de traction, une première extrémité du fil 37 étant reliée à l'aiguille 62 et une seconde extrémité du fil 37 étant reliée à l'instrument d'ancrage 8;
- une troisième étape d'accrochage amovible dudit organe 70 par l'instrument d'ancrage 8, l'instrument d'ancrage étant apte à accrocher successivement différents organes au cours de l'opération ;
- une quatrième étape de transpercement de l'intérieur vers l'extérieur de la paroi abdominale 52, à l'aide de l'aiguille 62 ou d'attache à un système d'ancrage intraabdominal transpariétal, puis de traction dudit organe 70 à l'aide de l'instrument d'ancrage 8 et du fil 37 ; et
- une cinquième étape de fixation de la première extrémité du fil 37 à l'extérieur de la paroi abdominale 52 à l'aide d'un moyen de fixation 76, pour former un point d'ancrage fixe 73, 79 de l'organe maintenu suspendu par le fil et l'instrument d'ancrage.

Ce procédé d'ancrage est réalisé durant une opération chirurgicale. Il est temporaire. Les organes maintenus suspendus par le fil et l'instrument d'ancrage sont décrochés à la fin de l'opération chirurgicale.
L'instrument d'ancrage selon l'invention, permet d'enserrer et de pincer les organes sans les percer ou les endommager.

## Revendications

1. Instrument d'ancrage pariétal (8) d'un organe (70) d'un être vivant, **caractérisé en ce qu'**il comprend:
- deux éléments longitudinaux (4, 6) articulés l'un à l'autre autour d'un axe d'articulation (10) médian pour former une pince (8), ladite pince (8) présentant de part et d'autre de l'axe d'articulation (10) d'une part des moyens (12, 16, 24) d'accrochage dudit organe, et d'autre part des moyens (14, 22, 28, 38) de préhension et de manipulation desdits moyens d'accrochage par une pince chirurgicale,
- les moyens de préhension (14, 22, 28, 38) comprenant deux disques d'appui (22, 28) chacun solidaire d'un élément longitudinal (4, 6) et muni d'une protubérance (38) sur sa surface externe pour faciliter la préhension de l'instrument par une pince chirurgicale, la protubérance (38) portée par chaque disque d'appui comprenant une partie d'extrémité (40) tronconique et une base (41) cylindrique solidaire dudit disque d'appui; et
- des moyens (36) d'attache d'au moins un élément (37) de traction auxdits moyens d'accrochage, l'élément de traction étant propre à être fixé à une paroi abdominale (52) dudit être vivant pour soulever et maintenir en suspension ledit organe (70) accroché par les moyens d'accrochage (12, 16, 24) pour exposer une structure anatomique au cours d'une opération chirurgicale en laparoscopie ou en coelioscopie,
les dimensions de l'instrument étant choisies pour permettre son passage à travers un trocart (53, 54) de laparoscopie ou de coelioscopie.

2. Instrument selon la revendication 1, **caractérisé en ce qu'**il comprend en outre un ressort (35) fixé à au moins un élément longitudinal (4, 6) et sollicitant lesdits moyens d'accrochage (12, 16, 24) vers une position de repos fermée.

3. Instrument selon la revendication 1 ou 2, **caractérisé en ce que** les moyens (36) d'attache d'un élément de traction sont formés au niveau de l'axe d'articulation (10) reliant les deux éléments longitudinaux (4, 6).

4. Instrument selon l'une quelconque des revendications 1 à 3, **caractérisé en ce que** les moyens (36) d'attache d'un élément de traction comprennent un élément qui définit un passage fermé.

5. Instrument selon la revendication 4, **caractérisé en ce que** ledit élément (36) est fixé à une partie de l'instrument (8) et forme une saillie par rapport à celle-ci.

6. Instrument selon la revendication 4, **caractérisé en ce que** ledit élément qui définit un passage formé comprend au moins un orifice traversant une partie de l'instrument (8).

7. Dispositif d'ancrage pariétal (58) d'un organe (70) d'un être vivant au cours d'une opération chirurgicale en laparoscopie ou en coelioscopie, **caractérisé en ce qu'**il comprend :
- au moins un élément de traction (37) propre à traverser la paroi abdominale (52) dudit être vivant ;
- des moyens (76) pour fixer extérieurement ledit élément de traction (37) à la paroi abdominale (52) dudit être vivant ; et
- un instrument d'ancrage pariétal (8) selon l'une quelconque des revendications précédentes, ledit élément de traction étant propre à être fixé d'une part aux moyens d'attache (36) dudit instrument (8) localisés à l'intérieur de la paroi abdominale (52), et d'autre part audit moyen (76) de fixation à l'extérieur de la paroi abdominale (52) pour former un point d'ancrage fixe (73, 79) de l'organe (70) maintenu suspendu par l'instrument d'ancrage (8) et l'élément de traction (37).

## Claims

1. Parietal anchoring instrument (8) for an organ (70) of a live patient, **characterised in that** it comprises:
- two longitudinal elements (4, 6) articulated to one another about a median articulation axis (10) to form a clamp (8), said clamp (8) having, on either side of the articulation axis (10), means (12, 16, 24) for attaching said organ, on the one hand, and means (14, 22, 28, 38) for gripping and manipulating said attachment means by a surgical clamp, on the other hand,
- said gripping means (14, 22, 28, 38) comprising two contact discs (22, 28) each fixedly connected to a longitudinal element (4, 6) and provided with a protrusion (38) on its outer surface to assist with the gripping of the instrument by a surgical clamp, the protrusion (38) carried by each contact disc comprising an end part (40) in the shape of a truncated cone and a cylindrical base (41) integral with the said contact disc; and
- means (36) for attaching at least one traction element (37) to said attachment means, the traction element being adapted to be fixed to an abdominal wall (52) of said live patient in order to raise and keep suspended said organ (70) attached by the attachment means (12, 16, 24) to expose an anatomical structure during a surgical laparoscopic or coelioscopic operation,
the dimensions of the instrument being selected so as to allow it to pass through a laparoscopic or coelioscopic trocar (53, 54).

2. Instrument according to claim 1, **characterised in that** it further comprises a spring (35) fixed to at least one longitudinal element (4, 6) and biasing said attachment means (12, 16, 24) towards a closed resting position.

3. Instrument according to claim 1 or 2, **characterised in that** the means (36) for attaching a traction element are formed level with the articulation axis (10) connecting the two longitudinal elements (4, 6).

4. Instrument according to any one of claims 1 to 3, **characterised in that** the means (36) for attaching a traction element comprise an element that defines a closed passage.

5. Instrument according to claim 4, **characterised in that** the said element (36) is fixed to part of the instrument (8) and forms a projection relative to said part.

6. Instrument according to claim 4, **characterised in that** the said element that defines a closed passage comprises at least one orifice passing through part of the instrument (8).

7. Parietal anchoring tool (58) for an organ (70) in a live patient during a surgical laparoscopic or coelioscopic operation, **characterised in that** it comprises:
- at least one traction element (37) adapted to pass through the abdominal wall (52) of the live patient;
- means (76) for externally fixing said traction element (37) to the abdominal wall (52) of said live patient; and
- a parietal anchoring instrument (8) according to any one of the preceding claims, the said traction element being adapted to be fixed on the one hand to the attachment means (36) for said instrument (8) which are located inside the abdominal wall (52), and on the other hand to the said fixing means (76) outside the abdominal wall (52) in order to form a fixed anchoring point (73, 79) for the organ (70) that is held suspended by the anchoring instrument (8) and the traction element (37).

## Patentansprüche

1. Parietales Verankerungselement (8) für ein Organ (70) eines Lebewesens, **dadurch gekennzeichnet, dass** es umfasst:
- zwei longitudinale Elemente (4, 6), die aneinander um eine mittlere Gelenkachse (10) angelenkt sind, um eine Zange (8) zu bilden, wobei die Zange (8) beiderseits der Gelenkachse (10) einerseits Mittel (12, 16, 24) zum Verankern des Organs und andererseits Mittel (14, 22, 28, 38) zum Greifen und Manipulieren der Verankerungsmittel durch eine chirurgische Zange aufweist,
- wobei die Greifmittel (14, 22, 28, 38) zwei Abstützscheiben (22, 28) aufweisen, die jeweils mit einem longitudinalen Element (4, 6) fest verbunden sind und auf ihrer äußeren Oberfläche mit einer Erhebung (38) versehen sind, um das Greifen des Instruments mit einer chirurgischen Zange zu erleichtern, wobei die Erhebung (38), die jede Abstützscheibe trägt, einen kegelstumpfförmigen Endabschnitt (40) und eine mit der Abstützscheibe fest verbundene zylindrische Basis (41) aufweist; und
- Mittel (36) zum Befestigen wenigstens eines Zugelements (37) an den Verankerungsmitteln, wobei das Zugelement dazu ausgelegt ist, an einer Abdominalwand (52) des Lebewesens befestigt zu werden, um das Organ (70), das durch die Verankerungsmittel (12, 16, 24) verankert ist, anzuheben und hängend zu halten, um eine anatomische Struktur während einer chirurgischen Laparoskopie- oder Coelioskopieoperation freizulegen,
wobei die Abmessungen des Instruments so gewählt sind, dass seine Hindurchführung durch einen Laparoskopie- oder Coeleoskopietrokar möglich ist.

2. Instrument nach Anspruch 1, **dadurch gekennzeichnet, dass** es außerdem eine Feder (35) umfasst, die an wenigstens einem longitudinalen Element (4, 6) befestigt ist und die Verankerungsmittel (12, 16, 24) in eine geschlossene Ruheposition drängt.

3. Instrument nach Anspruch 1 oder 2, **dadurch gekennzeichnet, dass** die Mittel (36) zum Befestigen eines Zugelements auf Höhe der Gelenkachse (10), die die zwei longitudinalen Elemente (4, 6) verbindet, gebildet sind.

4. Instrument nach einem der Ansprüche 1 bis 3, **dadurch gekennzeichnet, dass** die Mittel (36) zum Befestigen eines Zugelements ein Element enthalten, das einen geschlossenen Durchlass definiert.

5. Instrument nach Anspruch 4, **dadurch gekennzeichnet, dass** das Element (36) an einem Teil des Instruments (8) befestigt ist und in Bezug auf diesen einen Vorsprung bildet.

6. Instrument nach Anspruch 4, **dadurch gekennzeichnet, dass** das Element, das einen geschlossenen Durchlass definiert, wenigstens eine Öffnung auf weist, die durch einen Teil des Instruments (8) verläuft.

7. Parietale Verankerungsvorrichtung (58) für ein Organ (70) eines Lebewesens während einer chirurgischen Laparoskopie- oder Coelioskopieoperation, **dadurch gekennzeichnet, dass** sie umfasst:
- wenigstens ein Zugelement (37), das dazu ausgelegt ist, durch die Abdominalwand (52) des Lebewesens zu verlaufen;
- Mittel (76), um das Zugelement (37) an der Abdominalwand (52) des Lebewesens außen zu befestigen; und
- ein parietales Verankerungselement (8) nach einem der vorhergehenden Ansprüche, wobei das Zugelement dazu ausgelegt ist, einerseits an Mitteln (36) zum Befestigen des Instruments (8), die sich innerhalb der Abdominalwand (52) befinden, und andererseits an dem Mittel (76) zum Befestigung an der Außenseite der Abdominalwand (52) befestigt zu werden, um einen festen Verankerungspunkt (73, 79) des Organs (70) zu bilden, das durch das Verankerungselement (8) und das Zugelement (37) hängend gehalten wird.
